# EUROPÄISCHE PATENTSCHRIFT

(11) **EP 2 729 462 B1**
(45) Veröffentlichungstag und Bekanntmachung des Hinweises auf die Patenterteilung: **04.10.2017**
(21) Anmeldenummer: 12735264.9
(22) Anmeldetag: 05.07.2012
(51) Int. Cl.: C07D 401/14, A01N 43/713

(54) **VERFAHREN ZUR HERSTELLUNG VON TETRAZOL-SUBSTITUIERTEN ANTHRANILSÄUREDIAMID-DERIVATEN DURCH UMSETZUNG VON PYRAZOLSÄUREN MIT ANTHRANILSÄUREESTERN**
METHOD FOR MANUFACTURING TETRAZOLE SUBSTITUTED ANTHRANILIC ACID DERIVATIVES BY CONVERTING PYRAZOLE ACIDS WITH ANTHRANILIC ACID ESTERS
PROCÉDÉS DE FABRICATION DE DÉRIVÉS DE DIAMIDE D'ACIDE ANTHRANILIQUE SUBSTITUÉS PAR TÉTRAZOL, PAR RÉACTION DE L'ACIDE PYRAZOLIQUE AVEC DES ESTERS D'ACIDE ANTHRANILIQUE

(30) Priorität: 08.07.2011 EP 11173325; 11.07.2011 US 201161506265 P
(43) Veröffentlichungstag der Anmeldung: 14.05.2014
(73) Patentinhaber: Bayer Intellectual Property GmbH, 40789 Monheim (DE)
(72) Erfinder: PAZENOK, Sergii, 42699 Solingen (DE); VOLZ, Frank, 50825 Köln (DE); LUI, Norbert, 51519 Odenthal (DE); NEEFF, Arnd, 51399 Burscheid (DE); SZYWALSKI, Sylvia, 51377 Leverkusen (DE)
(74) Vertreter: BIP Patents
(86) Internationale Anmeldenummer: PCT/EP2012/063169
(87) Internationale Veröffentlichungsnummer: WO 2013/007604

(56) Entgegenhaltungen:
- WO-A2-2010/069502
- WO-A2-2011/098408

## Beschreibung

Die vorliegende Erfindung betrifft ein Verfahren zur Herstellung von tetrazol-substituierten Anthranilsäurediamid-Derivaten der Formel (I) 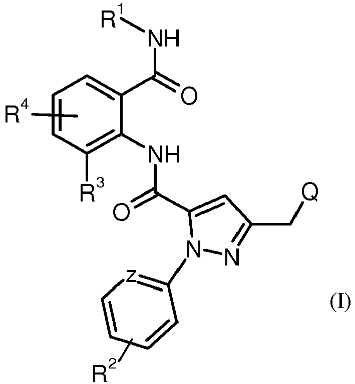 durch Umsetzung von N-aryl und N-hetarylsubstituierten Pyrazolsäuren, welche Methylentetrazolreste enthalten, mit Anthranilsäureestern und Aminen.

In der Literatur wurde bereits beschrieben, dass tetrazol-substituierte Anthranilsäurediamid-Derivate durch Umsetzung von tetrazol-substituierten N-aryl- und N-hetarylsubstituierten Pyrazolsäuren mit Anthranilsäureamiden hergestellt werden können (vgl. WO2010/069502). Ebenfalls können tetrazol-substituierte Anthranilsäurediamid-Derivate durch Umsetzung von tetrazol-substituierten Benzoxazinonen, mit Aminen erhalten werden (WO 2010/069502). Beide Verfahrens liefern eine gute, teilweise jedoch nur mäßige Ausbeute, insbesondere kann der Anteil an Regioisomeren, wobei der Tetrazolring Q in zwei unterschiedlichen Positionen angebunden ist, variieren. Die Aufgabe der vorliegenden Erfindung ist daher die Bereitstellung neuer, wirtschaftlicher Verfahren zur Herstellung von tetrazol-substituierten Anthranilsäurediamid-Derivaten der Formel (I) in höherer Reinheit und hoher Qualität, welche insbesondere ein konstantes Verhältnis der beiden möglichen Regioisomere liefern.

Die Aufgabe wurde gemäß der vorliegenden Erfindung gelöst durch ein Verfahren zur Herstellung von Anthranilsäurediamid-Derivaten der allgemeinen Formel (I), in welchen Q für Q-1 steht zu Verbindungen der Formel (I), in welchen Q für Q-2 steht, 90:10 bis 96:4 beträgt und wobei die Verbindungen der Formel (I) eine Reinheit > 90 % aufweisen, 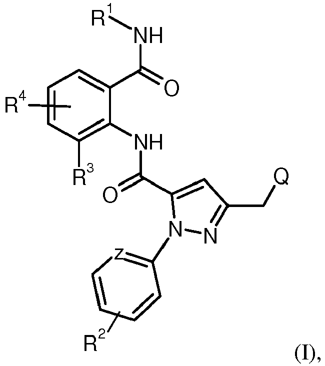 in welcher
- R¹,R³: unabhängig voneinander für (C₁-C₅)-Alkyl steht,
- R²: für Halogen oder C₁-C₆-Alkyl steht,
- R⁴: für Wasserstoff, Chlor oder Cyano steht,
- Q: für einen durch R⁵ einfach substituierten Tetrazolring steht, ausgewählt aus der Gruppe bestehend aus 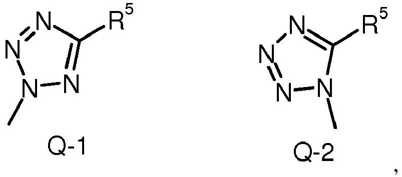
- R⁵: für (C₁-C₃)Perfluoralkyl steht,
- Z: für N steht,
die Verbindungen der allgemeinen Formel (I) außerdem N-Oxide und Salze umfassen,
dadurch gekennzeichnet, dass man tetrazol-substituierte Pyrazolsäuren der Formel (II) 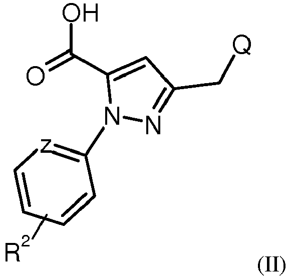 in welcher R², Q und Z oben angegebenen Bedeutungen haben
mit Anthranilsäureestern der Formel (III) 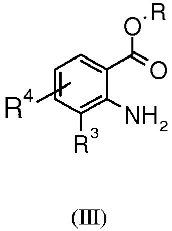 in welcher
- R: für Alkyl, Cycloalkyl, Alkoxyalkyl, Arylalkyl, Thioalkyl, Alkylthioalkyl, Alkylsulfonylalkyl, Cyanoalkyl, Haloalkyl, Nitroalkyl oder Aryl steht,
R³,R⁴ die oben angegebenen Bedeutungen haben,
zu Verbindungen der Formel (IV) umsetzt 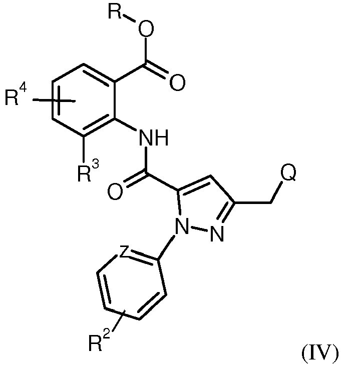 in welcher R, R², R³, R⁴, Q und Z oben angegebenen Bedeutungen haben
und diese Verbindungen der allgemeinen Formel (IV) mit Aminen der allgemeinen Formel (V)

R¹NH₂ (V)

in welcher R¹ die oben angegebenen Bedeutungen hat,
umsetzt zu Anthranilsäureamiden der Formel (I), 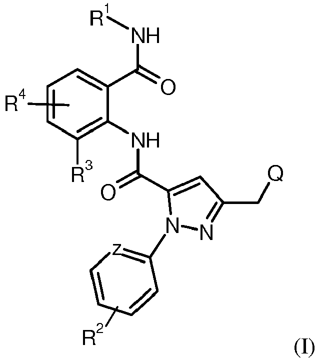 in welcher R¹, R², R³, R⁴, Q und Z die oben angegebenen Bedeutungen haben.

Durch das erfinderische Verfahren werden die Verbindungen der Formel (I) mit einer Reinheit von > 90%, bevorzugt von 91 %-97 %, besonders bevorzugt von 95 % bis 97 % erhalten, wobei das IsomerenVerhältnis der beiden möglichen Regioisomeren mit 90:10 bis 96:4 (Hauptisomer A, wobei Q für Q-1 steht: Nebenisomer B, wobei Q für Q-2 steht) konstant bleibt.

### Hauptisomer A

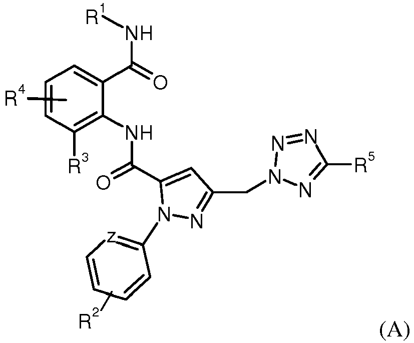

### Nebenisomer B

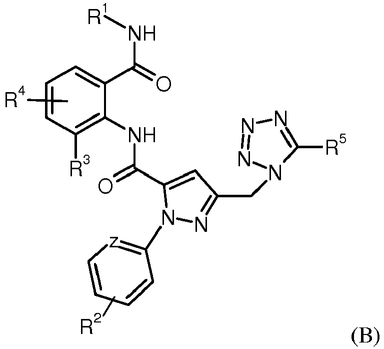

Das erfindungsgemäße Verfahren kann anhand des folgenden Schemas (I) erläutert werden : 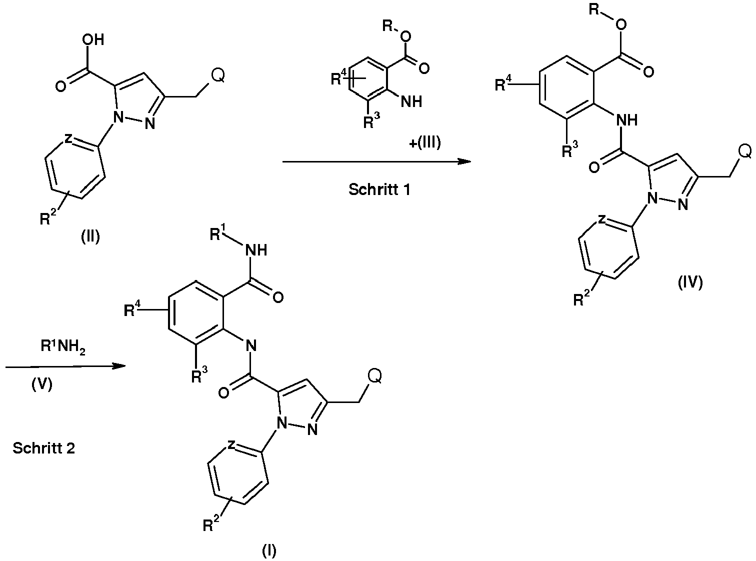 in welcher R, R¹, R², R³, R⁴, Q und Z die oben angegebenen allgemeinen Bedeutungen haben.,

### Schema (I)

### Allgemeine Definitionen:

Im Zusammenhang mit der vorliegenden Erfindung umfasst der Begriff Halogene (X), soweit nicht anders definiert, solche Elemente, die ausgewählt sind aus der Gruppe bestehend aus Fluor, Chlor, Brom und Jod, wobei Fluor, Chlor und Brom bevorzugt und Fluor und Chlor besonders bevorzugt verwendet werden. Substituierte Gruppen können einfach oder mehrfach substituiert sein, wobei bei Mehrfachsubstitutionen die Substituenten gleich oder verschieden sein können.

Mit einem oder mehreren Halogenatomen (-X) substituierte Alkyl-Gruppen (= Halogenalkyl-Gruppen) sind beispielsweise ausgewählt aus Trifluormethyl (CF₃), Difluormethyl (CHF₂), CCl₃, CFCl₂, CF₃CH₂, ClCH₂, CF₃CCl₂.

Alkyl-Gruppen sind im Zusammenhang mit der vorliegenden Erfindung, soweit nicht abweichend definiert, lineare oder verzweigte Kohlenwasserstoff-Gruppen. Alkylgruppen können im Zusammenhang mit der vorliegenden Erfindung ein- oder mehrfach durch weitere Gruppen substituiert sein, beispielsweise sind Cyanoalkyl-Gruppen ausgewählt aus Cyano-methyl, Cyano-ethyl, etc., Nitroalkylgruppen sind beispielsweise ausgewählt aus Nitro-methyl, Nitro-ethyl, etc.. Alkoxyalkylgruppen sind durch Alkoxy substitutierte Alkylgruppen, im einzelnen sind beispielsweise sind die Bedeutungen Methoxymethyl, Ethoxymethyl, Propoxyethyl, etc. umfasst.

Die Definition Alkyl und C₁-C₁₂-Alkyl umfasst beispielsweise die Bedeutungen Methyl, Ethyl, n-, isoPropyl, n-, iso-, sec- und t-Butyl, n-Pentyl, n-Hexyl, 1,3-Dimethylbutyl, 3,3-Dimethylbutyl, n-Heptyl, n-Nonyl, n-Decyl, n-Undecyl, n-Dodecyl.

Cycloalkyl-Gruppen sind im Zusammenhang mit der vorliegenden Erfindung, soweit nicht abweichend definiert, ringförmige gesättigte Kohlenwasserstoff-Gruppen.

Aryl-Reste sind im Zusammenhang mit der vorliegenden Erfindung, soweit nicht abweichend definiert, aromatische Kohlenwasserstoff-Reste, die ein, zwei oder mehrere Heteroatome, die ausgewählt sind aus O, N, P und S aufweisen können und optional durch weitere Gruppen substituiert sein können.

Arylalkyl-Gruppen und Arylalkoxy-Gruppen sind im Zusammenhang mit der vorliegenden Erfindung, soweit nicht abweichend definiert, durch Aryl-Gruppen substituierte Alkyl- bzw. Alkoxy-Gruppen, die eine Alkylenkette aufweisen können. Im Einzelnen umfasst die Definition Arylalkyl beispielsweise die Bedeutungen Benzyl- und Phenylethyl-; die Definition Arylalkoxy beispielsweise die Bedeutung Benzyloxy.

Alkylaryl-Gruppen (Alkaryl-Gruppen) und Alkylaryloxy-Gruppen sind im Zusammenhang mit der vorliegenden Erfindung, soweit nicht abweichend definiert, durch Alkyl-Gruppen substituierte Aryl-Gruppen, bzw Aryloxy-Gruppen, die eine C₁₋₈-Alkylenkette aufweisen können und im Arylgerüst oder Aryloxygerüst ein oder mehrere Heteroatome, die ausgewählt sind aus O, N, P und S, aufweisen können.

Die erfindungsgemäßen Verbindungen können gegebenenfalls als Mischungen verschiedener möglicher isomerer Formen, insbesondere von Stereoisomeren, wie z.B. E- und Z-, threo- und erythro-, sowie optischen Isomeren, gegebenenfalls aber auch von Tautomeren vorliegen. Es werden sowohl die E- als auch die Z-Isomeren, wie auch die threo- und erythro-, sowie die optischen Isomeren, beliebige Mischungen dieser Isomeren, sowie die möglichen tautomeren Formen offenbart

### Schritt 1.

Die Verbindungen der Formel (IV) werden durch Umsetzung von tetrazolsubstituierten Pyrazolsäuren der Formel (II) mit Anthranilsäureestern der Formel (III) erhalten.

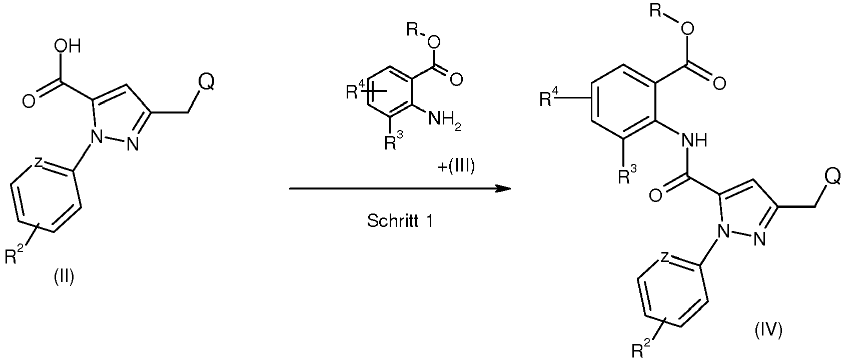

Anthranilsäureester der Formel (III) sind bekannt (vgl. WO 2008/070158). Pyrazolsäuren der Formel (II) sind ebenfalls bekannt (vgl. WO2007/144100). Pyrazolsäuren der Formel (II) können zum Beispiel aus Halogenmethylpyrazolestern der Formel (VI) und Perfluoralkyltetrazolen der Formel (VII) in zwei Schritten a und b hergestellt werden (vgl. Schema (II) und Herstellbeispiele). Dabei werden die gebildeten Verbindungen der Formel (VIII) durch basische Hydrolyse (Schritt b) in die Pyrazolsäuren der Formel (II) umgesetzt.

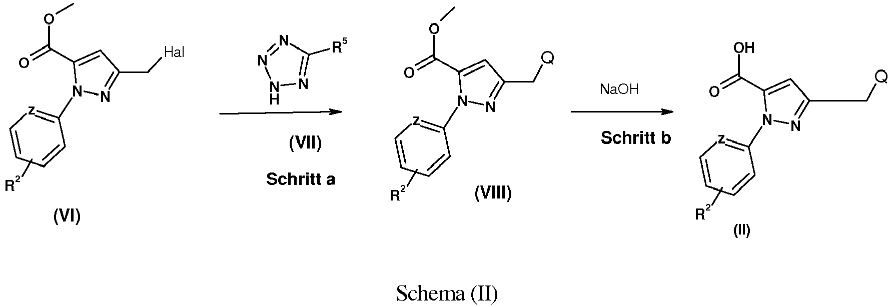

Halogenmethylpyrazolester der Formel (VI) sind ebenfalls bekannt und können wie in WO 2011/7073101 beschrieben hergestellt werden. Perfluoralkyltetrazole der Formel (VII) sind bekannt, teilweise sogar kommerziell erhältlich oder können nach bekannten Verfahren erhalten werden (vgl. z.B. WO2004/020445; William P. Norris, J. Org. Chem., 1962, 27 (9), 3248-3251; Henry C. Brown, Robert J. Kassal, J. Org. Chem., 1967, 32 (6), 1871-1873; Dennis P. Curran, Sabine Hadida, Sun-Young Kim, Tetrahedron, 1999, 55 (29), 8997-9006; L.D. Hansen, E.J. Baca, P. Scheiner, Journal of Heterocyclic Chemistry, 1970, 7, 991-996, JACS V.27, p.3248.

### Schritt 1

Schritt 1 wird grundsätzlich in Gegenwart einer Base durchgeführt. Geeignete Basen sind z. B. Natriumhydroxid, Kaliumcarbonat, Natriumcarbonat, Cäsiumcarbonat, Natriummethylat. Bevorzugt sind organischen Basen wie Trialkylamine, Pyridine, Alkylpyridine, Phosphazene und 1,8-Diazabicyclo[5.4.0]undecen (DBU). Besonders bevorzugt sind Pyridine, Alkylpyridine wie β-Picoline, 2,6-Dimethylpyridin, 2-Methyl-5-ethylpyridin, 2,3-Dimethylpyridin. Bei der Durchführung des erfindungsgemäßen Verfahrensschritts 1 setzt man für 1 Mol des Pyrazoles der Formel (II) vorzugsweise 1,5 Mol bis 4 Mol, besonders bevorzugt 1,5 bis 3 Equivalente der Base ein. Schritt 1 wird in Gegenwart eines Kondensationsmittels durchgeführt. Hierzu eignen sich alle für solche Kupplungsreaktionen üblichen Mittel. Beispielhaft genannt seien Säurehalogenidbildner wie Phosgen, Phosphortribromid, Phosphortrichlorid, Phosphorpentachlorid, Phosphoroxychlorid oder Thionylchlorid; Anhydridbildner wie Chlorameisensäureethylester, Chlorameisensäuremethylester, Chlorameisensäureisopropylester, Chlorameisensäureisobutylester oder Methansulfonylchlorid, p-Toluolsulfonsäure chlorid; Carbodiimide, wie N,N'-Dicyclohexylcarbodiimid (DCC) oder andere übliche Kondensationsmittel, wie Phosphorpentoxid, Polyphosphorsäure, 1,1'-Carbonyldiimidazol, 2-Ethoxy-N-ethoxycarbonyl-1,2-dihydrochinolin (EEDQ), Triphenylphosphin/Tetrachlorkohlenstoff, Brom-tripyrrolidinophosphonium-hexafluorophosphat, Bis(2-oxo-3-oxazolidinyl)phosphinchlorid oder Benzotriazol-1-yloxytris(dimethylamino)-phosphonium-hexafluorphosphat. Auf Polymer gestützte Reagenzien, wie z.B. polymer-gebundenes Cyclohexylcarbodiimid, können ebenfalls verwendet werden. Besonders geeignet sind Methansulfonylchlorid (Mesylchlorid) und Phosgen. Bei der Durchführung des erfindungsgemäßen Verfahrensschritts 1 setzt man für 1 Mol des Pyrazoles der Formel (II) vorzugsweise 1 Mol bis 3 Mol, besonders bevorzugt 1,5 bis 2,5 Mol der Kondensationsmittels ein.

Die Durchführung des erfindungsgemäßen Verfahrensschritts erfolgt vorzugsweise innerhalb eines Temperaturbereichs von 0°C bis +80°C, besonders bevorzugt bei Temperaturen von 10°C bis +50 °C.

Bei der Durchführung des erfindungsgemäßen Verfahrensschritts setzt man für 1 Mol der Pyrazolsäure der Formel (II) eine äquimolare Menge der Verbindung der Formel (III) ein.

Der erfindungsgemäße Verfahrensschritt (1) wird im Allgemeinen unter Normaldruck durchgeführt. Alternativ ist es jedoch auch möglich, im Vakuum zu arbeiten oder unter Druck.

Die Reaktionszeit ist nicht kritisch und kann, in Abhängigkeit von der Ansatzgröße, vom Substituent R⁵ und von der Temperatur, in einem Bereich zwischen ein und mehreren Stunden gewählt werden.

Geeignete Lösungsmittel sind beispielsweise aliphatische, alicyclische oder aromatische Kohlenwasserstoffe, wie z.B. Petrolether, n-Hexan, n-Heptan, Cyclohexan, Methylcyclohexan, Benzol, Toluol, Xylol oder Decalin, und halogenierte Kohlenwasserstoffe, wie z.B. Chlorbenzol, Dichlorbenzol, Dichlormethan, Chloroform, Tetrachlormethan, Dichlorethan oder Trichlorethan, Ether, wie Diethylether, Diisopropylether, Methyl-tert-butylether, Methyl-tert-amylether, Dioxan, Tetrahydrofuran, 1,2-Dimethoxyethan, 1,2-Diethoxyethan oder Anisol; Nitrile, wie Acetonitril, Propionitril, n- oder iso-Butyronitril oder Benzonitril; Amide, wie N,N-Dimethylformamid, N,N-Dimethylacetamid, N-Methylformanilid, N-Methylpyrrolidon oder Hexamethylphosphorsäuretriamid; Sulfoxide, wie Dimethylsulfoxid oder Sulfone, wie Sulfolan, Alkohole wie Methanol, Ethanol, Isopropanol oder Lösungsmittelmischungen. Besonders bevorzugt verwendet man Aceton, Acetonitril, Toluol, Methyl-tert.butylether, THF. Besonders geeignet sind Acetonitril, THF, DMF und NMP.

### Schritt 2

Die im Schritt 1 gebildeten Verbindungen der Formel (IV) werden zu Anthranilsäurediamid-Derivaten der Formel (I) umgesetzt: 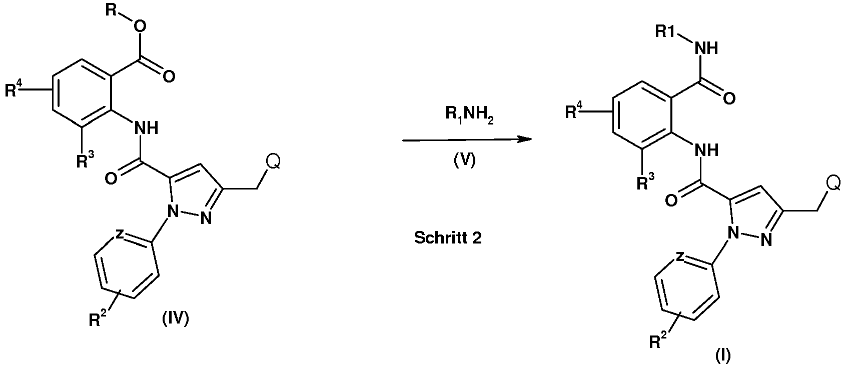 Überraschenderweise wurde nun gefunden dass die Verbindungen der Formel (IV) selektiv und unter sehr milden Bedingungen zu Anthranilsäurediamid-Derivaten der Formel (I) reagieren. Unter sehr milden Bedingungen sind beispielsweise, aber nicht limitierend, die folgenden Bedingungen zu verstehen:

Die Reaktion wird im Allgemeinen unter Normaldruck durchgeführt. Alternativ ist es jedoch auch möglich, unter Überdruck (z.B. Umsetzung im Autoklav mit MeNH₂) zu arbeiten.

Die Reaktionszeit kann, in Abhängigkeit von der Ansatzgröße und der Temperatur, in einem Bereich zwischen 1 Stunde und mehreren Stunden gewählt werden.

Der Reaktionsschritt wird vorzugweise in einem Lösungsmittel durchgeführt. Geeignete Lösungsmittel sind beispielsweise ausgewählt aus der Gruppe bestehend aus Wasser, Alkoholen wie Methanol, Ethanol, Isopropanol oder Butanol, aliphatischen und aromatischen Kohlenwasserstoffen, wie z.B. n-Hexan, Benzol oder Toluol, die durch Fluor- und Chloratome substituiert sein können, wie Methylenchlorid, Dichlorethan, Chlorbenzol oder Dichlorbenzol; Ethern, wie z.B. Diethylether, Diphenylether, Methyl-tert-butylether, Isopropylethylether, Dioxan, Diglym, Dimethylglycol, Dimethoxyethane (DME) oder THF; Nitrilen wie Methylnitril, Acetonitril, Butylnitril oder Phenylnitril; Amide wie Dimethylformamid (DMF) oder N-Methylpyrrolidon (NMP) oder Mischungen solcher Lösungsmittel, wobei Wasser, Acetonitril, Dichlormethan und Alkohole (Ethanol) besonders gut geeignet sind. Besonders bevorzugt sind THF, Acetonitril, Alkohole.

Man verwendet die Verbindungen der Formel (V) wobei R¹ bevorzugt für (C₁-C₆) Alkyl steht.

Der Austausch kann man zusätzlich durch Zugabe von Basen oder Säuren beschleunigen. Geeignete Basen sind Alkalimetallhydroxide wie z.B. Lithium-, Natrium- oder Kaliumhydroxid, Alkalimetallcarbonate wie beispielsweise Na₂CO₃, K₂CO₃ und -Acetate wie beispielsweise NaOAc, KOAc, LiO-Ac, sowie -Alkoholate, wie z.B. NaOMe, NaOEt, NaOt-Bu, KOt-Bu und organische Basen wie Trialkylamine, Alkylpyridine, Phosphazene und 1,8-Diazabicyclo[5.4.0]undecen (DBU). Bevorzugt sind die organische Basen wie Pyridine, Alkylpyridine .

Geeigneten Säuren sind CH₃COOH, CF₃COOH, p-TSA, HCl, H₂SO₄.

Die Durchführung des erfindungsgemäßen Verfahrensschritts (2) erfolgt vorzugsweise innerhalb eines Temperaturbereichs von 0°C bis +100°C, besonders bevorzugt bei Temperaturen von 10°C bis +80 °C, ganz besonders bevorzugt bei 10-60°C.

Der erfindungsgemäße Verfahrensschritt (2) wird im Allgemeinen unter Normaldruck durchgeführt. Alternativ ist es jedoch auch möglich, im Vakuum oder unter Überdruck im Autoklav zu arbeiten.

Die Reaktionszeit kann, in Abhängigkeit von der Ansatzgröße und der Temperatur, in einem Bereich zwischen 1 Stunde und mehreren Stunden gewählt werden.

### Herstellbeisniele

Die folgenden Herstellbeispiele illustrieren die Erfindung, ohne sie zu beschränken.

### Beispiel 1

Isomerengemisch von Methyl 1-(3-chloropyridin-2-yl)-3-{[5-(trifluoromethyl)-2H-tetrazol-2-yl]methyl}-1H-pyrazole-5-carboxylate (Hauptisomer) und Methyl 1-(3-chloropyridin-2-yl)-3-{[5-(trifluoromethyl)-1H-tetrazol-1-yl]methyl}-1H-pyrazole-5-carboxylate (Nebenkomponente).2.86 g (0.01 mol) Methyl 3-(chlormethyl)-1-(3-chloropyridin-2-yl)-1H-pyrazole-5-carboxylate und 1,6 g (0.01 mol) Natrium 5-(trifluoromethyl)tetrazol-2-ide und 0.15 g KI wurden in 50 ml Aceton 9 Std bei 56 °C erhitzt. Die Salze wurden abfiltriert und Aceton in Vakuum entfernt. Man erhielt 4.59 g des Produktes als 9:1 Gemisch von beiden Isomeren.

### Analytische Charakterisierung

### Hauptisomer

¹H NMR (CD₃CN) δ: 8,52 (1H, d); 7.95 (1H,d), 7,45 (1H, dd); 7,10 (1H, s); 6.05 (2H,s); 3,75 (3H, s) ppm.
¹⁹_{F} NMR -64.05 ppm.

### Nebenkomponente

¹⁹F NMR -61.46 ppm.
¹H NMR (CD₃CN) δ: 8,50 (1H, d); 7.90 (1H,d), 7,45 (1H, dd); 6,95 (1H, s); 5,80 (2H,s); 3,70 (3H, s) ppm.

### Beispiel 2

Isomerengemisch von 1-(3-chloropyridin-2-yl)-3-{[5-(trifluoromethyl)-2H-tetrazol-2-yl]methyl}-1H-pyrazole-5-carboxyl-Säure (Hauptisomer) und 1-(3-Chloropyridin-2-yl)-3-[5-(trifluoromethyl)-1H-tetrazol-1-yl]-1H-pyrazole-5-carboxyl-Säure (Nebenkomponente)

4.59 g des Gemisches aus dem Beispiel 1 wurden in 40 ml Methanol gelöst und 2 g NaOH als 10 % Lösung in Wasser wurden zugegeben. Das Gemisch wurde 3 Std. bei RT gerührt.

10 % iger HCl wurde zugegeben, um den pH-Wert der Lösung auf 3 einzustellen und das Produkt wurde mit Methyltert-butylether extrahiert. Nach der Entfernung des Lösemittels wird der Rückstand (4 g) ohne Aufreinigung weiter umgesetzt.

### Analytische Charakterisierung Hauptisomer 92 %

¹H NMR (CD₃CN) δ: 13,5 (b.s); 8,52 (1H, d); 8,2 (1H,d), 7,6 (1H, dd); 7,2 (1H, s); 6.25 (2H,s) ppm.
¹⁹F NMR -64.25 ppm.

### Beispiel 3

Isomerengemisch von Methyl 2-({[1-(3-chloropyridin-2-yl)-3-{[5-(trifluoromethyl)-2H-tetrazol-2-yl]methyl}-1H-pyrazol-5-yl]carbonyl}amino)-5-cyano-3-methylbenzoate und methyl 2-({[1-(3-chloropyridin-2-yl)-3-[5-(trifluoromethyl)-1H-tetrazol-1-yl]methyl}-1H-pyrazol-5-yl]carbonyl}amino)-5-cyano-3-methylbenzoate

3,73 g (10 mmol) des Gemisches von 1-(3-chloropyridin-2-yl)-3-{[5-(trifluoromethyl)-2H-tetrazol-2-yl]methyl}-1H-pyrazole-5-carboxyl-Säure und 1-(3-Chloropyridin-2-yl)-3-[5-(trifluoromethyl)-1H-tetrazol-1-yl]-1H-pyrazole-5-carboxyl-Säure im Verhältnis 9:1 wurden in 20 ml Acetonitrile vorgelegt, auf 0°C gekühlt und bei dieser Temperatur zuerst 1,97 g (27 mmol) Pyridin und dann 1,93 g (17 mmol) Methansulfonsäurechlorid zugegeben. Das Gemisch wurde 1 Std bei 0°C gerührt und anschließend bei 0°C 1,9 g (10 mmol) Methyl 2-amino-5-cyano-3-methylbenzoate und 0,79 g (10 mmol) Pyridin zugegeben.

Das Reaktionsgemisch wurde auf 50°C erhitzt und insgesamt 12 h bei 50°C nachgerührt. 30 ml Wasser wurden zugegeben und das Gemisch auf 10°C abgekühlt. Der Niederschlag wurde abfiltriert und mit Wasser gewaschen. Man erhielt 4,63 g (85 %) des Produktes als Gemisch von beiden Regioisomeren im Verhältnis 93:7.

**Analytische Charakterisierung:**

| H/C | δH/ppm | Mult. | rel. No. H | δH/ppm Isomer | Mult. | rel. No. H |
|---|---|---|---|---|---|---|
| 1 | 8.00 | D | 1 | 8.00 | D | 1 |
| 2 | 8.01 | D | 1 | 8.01 | D | 1 |
| 3 | 3.70 | S | 3 | 3.70 | S | 3 |
| 4 | 10.58 | S | 1 | 10.57 | S | 1 |
| 5 | 2.26 | S | 3 | 2.25 | S | 3 |
| 6 | 7.42 | S | 1 | 7.36 | S | 1 |
| 7 | 6.34 | S | 2 | 6.12 | S | 2 |
| 8 | 8.49 | DD | 1 | 8.48 | DD | 1 |
| 9 | 7.61 | DD | 1 | 7.60 | DD | 1 |
| 10 | 8.18 | DD | 1 | 8.15 | DD | 1 |

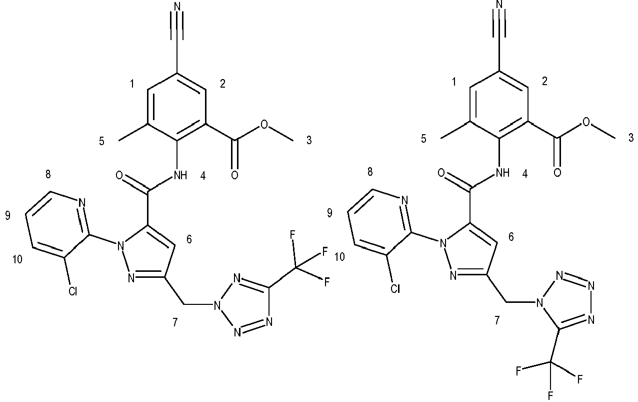

### Beispiel 4

Isomerengemisch von Ethyl 2-({[1-(3-chloropyridin-2-yl)-3-{[5-(trifluoromethyl)-2H-tetrazol-2-yl]methyl}-1H-pyrazol-5-yl]carbonyl}amino)-5-cyano-3-methylbenzoate und Ethyl 2-({[1-(3-chloropyridin-2-yl)-3-{[5-(trifluoromethyl)-1H-tetrazol-1-yl]methyl}-1H-pyrazol-5-yl]carbonyl}amino)-5-cyano-3-methylbenzoate

Man arbeitet wie in Beispiel 1 beschrieben, nimmt jedoch Ethyl 2-amino-5-cyano-3-methylbenzoate. Ausbeute 81 %.

### Beispiel 5

Isomerengemisch von 1-(3-chloropyridin-2-yl)-N-[4-cyano-2-methyl-6-(methylcarbamoyl)-phenyl]-3-{[5-(trifluoromethyl)-2H-tetrazol-2-yl]methyl}-1H-pyrazole-5-carboxamide (Hauptisomer) und 1-(3-chloropyridin-2-yl)-N-[4-cyano-2-methyl-6-(methylcarbamoyl)phenyl]-3-[5-(trifluoro-methyl)-1H-tetrazol-1-yl]-1H-pyrazole-5-carboxamide (Nebenkomponente) im Verhältnis 93:7.

5,45 g des Isomerengemisches von Methyl 2-({[1-(3-chloropyridin-2-yl)-3-{[5-(trifluoromethyl)-2H-tetrazol-2-yl]methyl}-1H-pyrazol-5-yl]carbonyl}amino)-5-cyano-3-methylbenzoate und Methyl 2-({[1-(3-chloropyridin-2-yl)-3-{[5-(trifluoromethyl)-1H-tetrazol-1-yl]methyl}-1H-pyrazol-5-yl]carbonyl}amino)-5-cyano-3-methylbenzoate wurden in 30 ml Acetonitril gelöst. Dann wurde 1 Äquivalent Methylamin (als Lösung in THF) zugegeben. Das Gemisch wurde 4 Std bei 30°C nachgerührt, mit 30 ml Wassser verdünnt und der Niederschlag abfiltriert. Man erhielt 5,1 g(93 %) des Produktes als weissen Feststoff mit einem Isomerenverhältnis von 93:7.

### Analytische Charakterisierung

**Hauptisomer 94 %**

| H/C | δH/ppm | Mult. | rel. No. H | δC/ppm | Mult. | rel. No. C |
|---|---|---|---|---|---|---|
| 1 | - | - | - | 118.7 | Q | 1 |
| 2 | - | - | - | 156.1 | Q | 1 |
| 3 | 6.34 | S | 2 | 51.3 | T | 1 |
| 4 | - | - | - | 145.6 | S | 1 |
| 5 | 7.40 | S | 1 | 108.5 | D | 1 |
| 6 | - | - | - | 138.8 | S | 1 |
| 7 | - | - | - | 156.3 | S | 1 |
| 8 | 10.55 | S | 1 | - | - | - |
| 9 | - | - | - | 137.6 | S | 1 |
| 10 | - | - | - | 138.7 | S | 1 |
| 11 | - | - | - | 166.2 | S | 1 |
| 12 | 8.38 | Q | 1 | - | - | - |
| 13 | 2.66 | D | 3 | 26.3 | Q | 1 |
| 14 | 7.75 | D | 1 | 129.7 | D | 1 |
| 15 | - | - | - | 109.4 | S | 1 |
| 16 | - | - | - | 118.3 | S | 1 |
| 17 | 7.87 | D | 1 | 135.2 | D | 1 |
| 18 | - | - | - | 138.0 | S | 1 |
| 19 | 2.20 | S | 3 | 18.0 | Q | 1 |
| 20 | - | - | - | 149.1 | S | 1 |
| 21 | - | - | - | 128.0 | S | 1 |
| 22 | 8.16 | DD | 1 | 139.4 | D | 1 |
| 23 | 7.60 | DD | 1 | 126.7 | D | 1 |
| 24 | 8.48 | DD | 1 | 147.3 | D | 1 |

**Nebenkomponente**

| H/C | δH/ppm | Mult. | rel. No. H | δC/ppm | Mult. | rel. No. C |
|---|---|---|---|---|---|---|
| 1 | - | - | - | 118.1 | Q | 1 |
| 2 | - | - | - | 145.9 | Q | 1 |
| 3 | 6.11 | S | 2 | 47.0 | T | 1 |
| 4 | - | - | - | 145.9 | S | 1 |
| 5 | 7.35 | S | 1 | 107.7 | D | 1 |
| 6 | - | - | - | 138.8 | S | 1 |
| 7 | - | - | - | 156.2 | S | 1 |
| 8 | 10.54 | S | 1 | - | - | - |
| 9 | - | - | - | 137.6 | S | 1 |
| 10 | - | - | - | 135.2 | S | 1 |
| 11 | - | - | - | 166.2 | S | 1 |
| 12 | 8.37 | Q | 1 | - | - | - |
| 13 | 2.66 | D | 3 | 26.3 | Q | 1 |
| 14 | 7.75 | D | 1 | 129.7 | D | 1 |
| 15 | - | - | - | 109.3 | S | 1 |
| 16 | - | - | - | 118.3 | S | 1 |
| 17 | 7.87 | D | 1 | 135.4 | D | 1 |
| 18 | - | - | - | 138.0 | S | 1 |
| 19 | 2.19 | S | 3 | 17.9 | Q | 1 |
| 20 | - | - | - | 149.1 | S | 1 |
| 21 | - | - | - | 128.1 | S | 1 |
| 22 | 8.14 | DD | 1 | 139.4 | D | 1 |
| 23 | 7.58 | DD | 1 | 126.7 | D | 1 |
| 24 | 8.47 | DD | 1 | 147.2 | D | 1 |

### Beispiel 6

Isomerengemisch von 1-(3-chloropyridin-2-yl)-N-[4-cyano-2-methyl-6-(methylcarbamoyl)-phenyl]-3-{[5-(trifluoromethyl)-2H-tetrazol-2-yl]methyl}-1H-pyrazole-5-carboxamide (Hauptisomer) und 1-(3-chloropyridin-2-yl)-N-[4-cyano-2-methyl-6-(methylcarbamoyl)phenyl]-3-[5-(trifluoro-methyl)-1H-tetrazol-l-yl]-1H-pyrazole-5-carboxamide (Nebenkomponente) im Verhältnis 93:7.

Man arbeitet wie in Beispiel 3 beschrieben, nimmt jedoch das Isomerengemisch von Ethyl 2-({[1-(3-chloropyridin-2-yl)-3-{[5-(trifluoromethyl)-2H-tetrazol-2-yl]methyl}-1H-pyrazol-5-yl]carbonyl}amino)-5-cyano-3-methylbenzoate und Ethyl 2-({[1-(3-chloropyridin-2-yl)-3-{[5-(trifluoromethyl)-1H-tetrazol-1-yl]methyl}-1H-pyrazol-5-yl]carbonyl}amino)-5-cyano-3-methylbenzoate.

## Patentansprüche

1. Verfahren zur Herstellung von Verbindungen der Formel (I), wobei Verhältnis von Verbindungen der Formel (I), in welchen Q für Q-1 steht zu Verbindungen der Formel (I), in welchen Q für Q-2 steht, 90:10 bis 96:4 beträgt und wobei die Verbindungen der Formel (I) eine Reinheit > 90 % aufweisen, 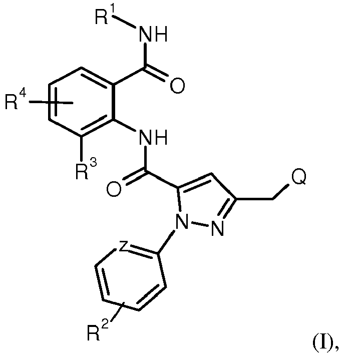 in welcher
R¹, R³ unabhängig voneinander für (C₁-C₅)-Alkyl steht,
R² für Halogen oder C₁-C₆-Alkyl steht,
R⁴ für Wasserstoff, Chlor oder Cyano steht,
Q für einen durch R⁵ einfach substituierten Tetrazolring steht, ausgewählt aus der Gruppe bestehend aus 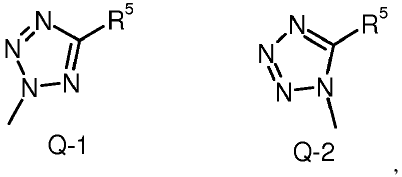
R⁵ für (C₁-C₃)Perfluoralkyl steht,
Z für N steht,
die Verbindungen der allgemeinen Formel (I) außerdem N-Oxide und Salze umfassen,
**dadurch gekennzeichnet, dass** man tetrazol-substituierte_Pyrazolsäuren der Formel (II) 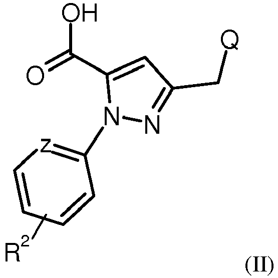 in welcher R², Q und Z die oben angegebenen Bedeutungen haben
mit Anthranilsäureestern der Formel (III) 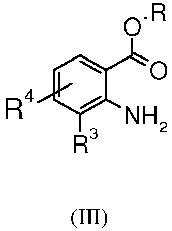 in welcher
R für Alkyl, Cycloalkyl, Alkoxyalkyl, Arylalkyl, Thioalkyl, Alkylthioalkyl, Alkylsulfonylalkyl, Cyanoalkyl, Haloalkyl, Nitroalkyl oder Aryl steht,
R³ ,R⁴ die oben angegebenen Bedeutungen haben,
unter Zugabe einer Base zu Verbindungen der Formel (IV) umsetzt, 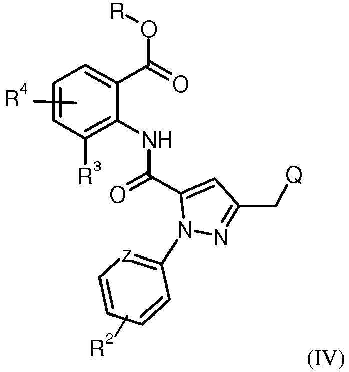 in welcher R, R², R³, R⁴, Q und Z die oben angegebenen Bedeutungen haben
und diese Verbindungen der allgemeinen Formel (IV) mit Aminen der allgemeinen Formel (V)
R¹NH₂ (V)
in welcher R¹ die oben angegebenen Bedeutungen hat,
umsetzt zu Anthranilsäureamiden der Formel (I).

2. Verfahren zur Herstellung von Verbindungen der Formel (I) gemäß Anspruch 1, **dadurch gekennzeichnet, dass**
R¹, R³ unabhängig voneinander für Methyl, Ethyl oder tert-Butyl steht,
R² für Fluor oder Chlor steht,
R⁴ für Chlor oder Cyano steht,
Q für Q-1 oder Q-2 steht,
R⁵ für CF₃ oder C₂F₅ steht,
Z für N steht.

3. Verfahren zur Herstellung von Verbindungen der Formel (I) gemäß einem der Ansprüche 1 bis 2, **dadurch gekennzeichnet dass** R⁵ für CF₃ steht.

4. Verfahren zur Herstellung von Verbindungen der Formel (I) gemäß einem der Ansprüche 1 bis 3, **dadurch gekennzeichnet, dass** R² für Chlor, R³ für Methyl und R⁴ für Cyano steht.

5. Verfahren zur Herstellung von Verbindungen der Formel (I) gemäß einem der Ansprüche 1 bis 4, **dadurch gekennzeichnet, dass** die Verbindungen der Formel (IV) bei einer Reaktionstemperatur von 0°C bis +100°C mit den Verbindungen der Formel (V) zu Anthranilsäurediamid-Derivaten der Formel (I) umgesetzt werden.

## Claims

1. Process for preparing compounds of the formula (I), where the ratio of compounds of the formula (I) in which Q represents Q-1 to compounds of the formula (I) in which Q represents Q-2 is from 90:10 to 96:4, 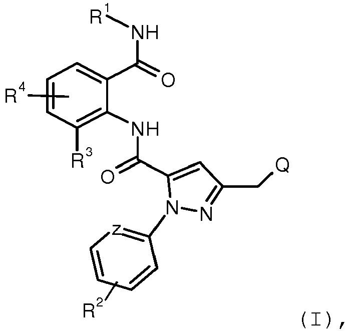 in which
R¹, R³ independently of one another represent (C₁-C₅)-alkyl,
R² represents halogen or C₁-C₆-alkyl,
R⁴ represents hydrogen, chlorine or cyano,
Q represents a tetrazole ring which is monosubstituted by R⁵ and selected from the group consisting of 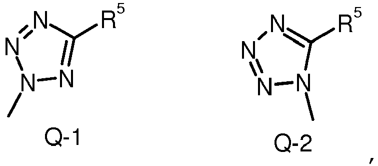
R⁵ represents (C₁-C₃)-perfluoroalkyl,
Z represents N,
the compounds of the general formula (I) furthermore include N-oxides and salts,
**characterized in that** tetrazole-substituted pyrazole acids of the formula (II) 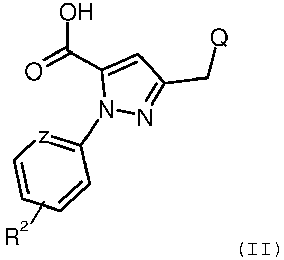 in which R², Q and Z have the meanings given above
are reacted with anthranilic esters of the formula (III) 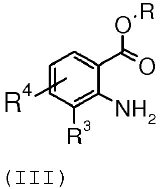 in which
R represents alkyl, cycloalkyl, alkoxyalkyl, arylalkyl, thioalkyl, alkylthioalkyl, alkylsulphonylalkyl, cyanoalkyl, haloalkyl, nitroalkyl or aryl,
R³, R⁴ have the meanings given above,
to give compounds of the formula (IV) with the addition of a base 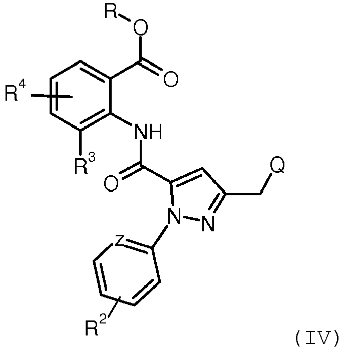 in which R, R², R³, R⁴, Q and Z have the meanings given above,
and these compounds of the general formula (IV) are reacted with amines of the general formula (V)
R¹NH₂ (V)
in which R¹ has the meanings given above,
to give anthranilamides of the formula (I).

2. Process for preparing compounds of the formula (I) according to Claim 1, **characterized in that**
R¹, R³ independently of one another represent methyl, ethyl or tert-butyl,
R² represents fluorine or chlorine,
R⁴ represents chlorine or cyano,
Q represents Q-1 or Q-2,
R⁵ represents CF₃ or C₂F₅,
Z represents N.

3. Process for preparing compounds of the formula (I) according to any of Claims 1 to 2, **characterized in that** R⁵ represents CF₃.

4. Process for preparing compounds of the formula (I) according to any of Claims 1 to 3, **characterized in that** R² represents chlorine, R³ represents methyl and R⁴ represents cyano.

5. Process for preparing compounds of the formula (I) according to any of Claims 1 to 4, **characterized in that** the compounds of the formula (IV) are reacted at a reaction temperature of from 0°C to +100°C with the compounds of the formula (V) to give anthranilic acid diamide derivatives of the formula (I).

## Revendications

1. Procédé de fabrication de composés de formule (I), le rapport entre les composés de formule (I) dans lesquels Q représente Q-1 et les composés de formule (I) dans lesquels Q représente Q-2 étant de 90:10 à 96:4, et les composés de formule (I) présentant une pureté > 90 %, 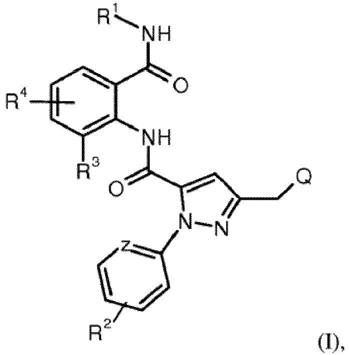 dans laquelle
R¹, R³ représentent indépendamment l'un de l'autre alkyle en (C₁-C₅),
R² représente halogène ou alkyle en C₁-C₆,
R⁴ représente hydrogène, chlore ou cyano,
Q représente un cycle tétrazole substitué une fois par R⁵, choisi dans le groupe constitué par 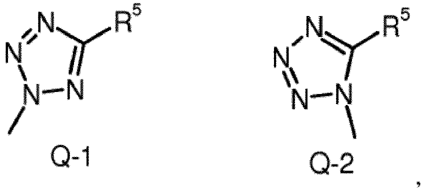
R⁵ représente perfluoroalkyle en (C₁-C₃),
Z représente N,
les composés de formule générale (I) comprenant en outre les N-oxydes et les sels,
**caractérisé en ce que** des acides pyrazoliques à substitution tétrazole de formule (II) 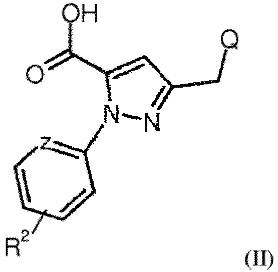 dans laquelle R², Q et Z ont les significations indiquées précédemment,
sont mis en réaction avec des esters de l'acide anthranilique de formule (III) 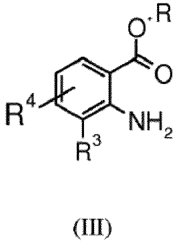 dans laquelle
R représente alkyle, cycloalkyle, alcoxyalkyle, arylalkyle, thioalkyle, alkylthioalkyle, alkylsulfonylalkyle, cyanoalkyle, haloalkyle, nitroalkyle ou aryle,
R³, R⁴ ont les significations indiquées précédemment, avec ajout d'une base pour former des composés de formule (IV) 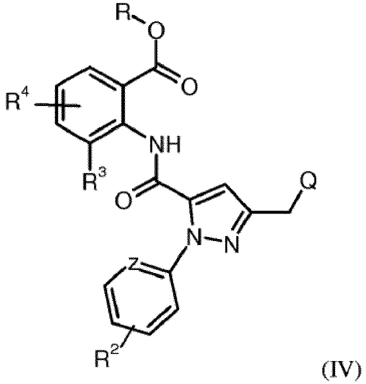 dans laquelle R, R², R³, R⁴, Q et Z ont les significations indiquées précédemment,
et ces composés de formule générale (IV) sont mis en réaction avec des amines de formule générale (V)
R¹NH₂ (V)
dans laquelle R¹ a les significations indiquées précédemment,
pour former des amides de l'acide anthranilique de formule (I).

2. Procédé de fabrication de composés de formule (I) selon la revendication 1, **caractérisé en ce que** R¹, R³ représentent indépendamment l'un de l'autre méthyle, éthyle ou tert-butyle,
R² représente fluor ou chlore,
R⁴ représente chlore ou cyano,
Q représente Q-1 ou Q-2,
R⁵ représente CF₃ ou C₂F₅,
Z représente N.

3. Procédé de fabrication de composés de formule (I) selon l'une quelconque des revendications 1 à 2, **caractérisé en ce que** R⁵ représente CF₃.

4. Procédé de fabrication de composés de formule (I) selon l'une quelconque des revendications 1 à 3, **caractérisé en ce que** R² représente chlore, R³ représente méthyle et R⁴ représente cyano.

5. Procédé de fabrication de composés de formule (I) selon l'une quelconque des revendications 1 à 4, **caractérisé en ce que** les composés de formule (IV) sont mis en réaction à une température de réaction de 0 °C à +100 °C avec les composés de formule (V) pour former des dérivés de diamide de l'acide anthranilique de formule (I).
